# EUROPEAN PATENT SPECIFICATION

(11) **EP 2 441 468 B1**
(45) Date of publication and mention of the grant of the patent: **08.01.2020**
(21) Application number: 11189797.1
(22) Date of filing: 21.02.2007
(51) Int. Cl.: A61K 39/00, C07K 14/705

(54) **Immunotherapy by using cell capable of co-expressing target antigen and cd1d and pulsed with cd1d ligand**
Immuntherapie mithilfe einer mit einem CD1d-Liganden gepulsten Zelle zur Co-Expression eines Ziel-Antigens und von CD1d
Immunothérapie par l'utilisation de cellules capable de co-exprimer l'antigène cible et le CD1d et impulsées par le ligand CD1d

(30) Priority: 22.02.2006 JP 2006045193
(43) Date of publication of application: 18.04.2012
(62) Divisional of application: 07714709.8
(73) Proprietor: Riken, Wako-shi, Saitama 351-0198 (JP)
(72) Inventor: Fujii, Shin-ichiro, Yokohama-shi, Kanagawa 230-0045 (JP); Shimizu, Kanako, Yokohama-shi, Kanagawa 230-0045 (JP)
(74) Representative: J A Kemp LLP

(56) References cited:
- WO-A2-2004/101776
- METELITSA L S ET AL: "Human NKT cells mediate antitumor cytotoxicity directly by recognizing target cell CD1d with bound ligand or indirectly by producing IL-2 to activate NK cells", JOURNAL OF IMMUNOLOGY, AMERICAN ASSOCIATION OF IMMUNOLOGISTS, US, vol. 167, no. 6, 15 September 2001 (2001-09-15), pages 3114-3122, XP003017208, ISSN: 0022-1767
- FAIS F ET AL: "CD1d is expressed on B-chronic lymphocytic leukemia cells and mediates alpha-galactosylceramide presentation to natural killer T lymphocytes", INTERNATIONAL JOURNAL OF CANCER, JOHN WILEY & SONS, INC, UNITED STATES, SWITZERLAND, GERMANY, vol. 109, no. 3, 10 April 2004 (2004-04-10), pages 402-411, XP003017210, ISSN: 0020-7136, DOI: 10.1002/IJC.11723
- LIU KANG ET AL: "Innate NKT lymphocytes confer superior adaptive immunity via tumor-capturing dendritic cells", THE JOURNAL OF EXPERIMENTAL MEDICINE, ROCKEFELLER UNIVERSITY PRESS, US, vol. 202, no. 11, 5 December 2005 (2005-12-05), pages 1507-1516, XP002534263, ISSN: 0022-1007, DOI: 10.1084/JEM.20050956
- Mark A. Exley ET AL: "CD1d-reactive T-cell activation leads to amelioration of disease caused by diabetogenic encephalomyocarditis virus", J. Leukoc. Biol., 1 May 2001 (2001-05-01), pages 713-718, XP55035143, Retrieved from the Internet: URL:http://www.jleukbio.org/content/69/5/7 13.full.pdf#page=1&view=FitH [retrieved on 2012-08-09]
- Emanuele Durante-Mangoni ET AL: "Hepatic CD1d Expression in Hepatitis CVirus Infection and Recognition by ResidentProinflammatory CD1d-Reactive T Cells", J Immunol, 1 August 2004 (2004-08-01), pages 2159-2166, XP55035141, Retrieved from the Internet: URL:http://www.jimmunol.org/content/173/3/ 2159.full.pdf#page=1&view=FitH [retrieved on 2012-08-09]
- KO S-Y ET AL: "alpha-galactosylceramide can act as a nasal vaccine adjuvant inducing protective immune responses against viral infection and tumor", THE JOURNAL OF IMMUNOLOGY, THE AMERICAN ASSOCIATION OF IMMUNOLOGISTS, US, vol. 175, no. 5, 1 September 2005 (2005-09-01), pages 3309-3317, XP002363110, ISSN: 0022-1767
- FUJII S-I ET AL: "Prolonged IFN-gamma-producing NKT response induced with alpha-galactosylceramide-loaded DCs", NATURE IMMUNOLOGY, NATURE PUBLISHING GROUP, GB, vol. 3, no. 9, 1 January 2002 (2002-01-01) , pages 867-874, XP003007246, ISSN: 1529-2908, DOI: 10.1038/NI827
- SILK J D ET AL: "Utilizing the adjuvant properties of CD1d-dependent NK T Cells in T cell-mediated immunotherapy", JOURNAL OF CLINICAL INVESTIGATION, AMERICAN SOCIETY FOR CLINICAL INVESTIGATION, US, vol. 114, no. 12, 1 December 2004 (2004-12-01), pages 1800-1811, XP003017211, ISSN: 0021-9738, DOI: 10.1172/JCI200422046
- IAN HERMANS ET AL: "NKT cells enhance CD4+ and CD8+ T cell responses to soluble antigen in vivo through direct interaction with dendritic cells.", THE JOURNAL OF IMMUNOLOGY, vol. 171, no. 10, 1 November 2003 (2003-11-01), pages 5140-5147, XP55020359, ISSN: 0022-1767
- CUI ET AL: "Requirement for Valpha14 NKT cells in IL-12-mediated rejection of tumors.", SCIENCE, vol. 278, no. 5343, 1 November 1997 (1997-11-01), pages 1623-1626, XP55020360, ISSN: 0036-8075
- SHIN-ICHIRO FUJII ET AL: "Activation of natural killer T cells by alpha-galactosylceramide rapidly induces the full maturation of dendritic cells in vivo and thereby acts as an adjuvant for combined CD4 and CD8 T cell immunity to a coadministered protein", THE JOURNAL OF EXPERIMENTAL MEDICINE, ROCKEFELLER UNIVERSITY PRESS, US, vol. 198, no. 2, 21 July 2003 (2003-07-21) , pages 267-279, XP002664689, ISSN: 0022-1007, DOI: 10.1084/JEM.20030324
- KAWANO T ET AL: "CD1d-restricted and TCR-mediated activation of V(alpha)14 NKT cells by glycosylceramides", SCIENCE, AMERICAN ASSOCIATION FOR THE ADVANCEMENT OF SCIENCE, WASHINGTON, DC; US, vol. 278, 1 January 1997 (1997-01-01), pages 1626-1629, XP002175772, ISSN: 0036-8075, DOI: 10.1126/SCIENCE.278.5343.1626
- GIACCONE G ET AL: "A phase I study of the natural killer T-cell ligand alpha-galactosylceramide (KRN7000) in patients with solid tumors", CLINICAL CANCER RESEARCH, THE AMERICAN ASSOCIATION FOR CANCER RESEARCH, US, vol. 8, no. 12, 1 December 2002 (2002-12-01), pages 3702-3709, XP003017212, ISSN: 1078-0432
- Hidetake Matsuyoshi ET AL: "Therapeutic effect of alpha-galactosylceramide-loaded dendritic cells genetically engineered to express SLC/CCL21 along with tumor antigen against peritoneally disseminated tumor cells", CANCER SCIENCE, vol. 96, no. 12, 1 December 2005 (2005-12-01), pages 889-896, XP55333357, JP ISSN: 1347-9032, DOI: 10.1111/j.1349-7006.2005.00123.x

## Description

### Technical Field

The present invention relates to a cell co-expressing target antigen and CD1d and having an ability to immunoactivate the target antigen, a method of producing it, an immunoinducer for a target antigen, and the like.

### Background Art

NKT cell is a cell constituting a lymphocyte lineage simultaneously having the characteristics of both T cell and NK cell, which is activated by an antigen present on MHC class I-like molecule CD1d, and shows actions such as an antitumor action and the like. Examples of the antigen (CD1d ligand) present on CD1d, which strongly activates NKT cells, include glycolipid α-galactosylceramide (α-GalCer). Primary NKT cell is stimulated by an antigen presenting cell (APC) loaded with an antigen such as α-GalCer and the like, and can differentiate · proliferate. Examples of such APC include macrophage, immature or mature dendritic cell (DC) and the like. Of these, the present inventors previously reported that mature DC pulsed with α-GalCer strongly activates NKT cells.

Heretofore, as an immunotherapy by NKT cell, administration of NKT cell activated by dendritic cell presenting CD1d ligand has clinically been tried. In addition, the present inventors reported that an antigen specific T cell immunity induction occurs as an adjuvant effect of a CD1d ligand by administration of the CD1d ligand to a mouse model.

Known literatures in the field of the invention by the present inventors include the following.
Non-patent reference 1 describes that, in a mouse model, a mature DC pulsed with α-GalCer can strongly activate IFN-γ-producing NKT cells, and that an NKT cell thus induced shows an antitumor effect.
Non-patent reference 2 describes that, in an experiment using a human sample, a mature DC pulsed with α-GalCer can strongly activate IFN-γ producing NKT cells, and that macrophage and immature dendritic cell can also activate NKT cells.
Non-patent reference 3 describes that when a dendritic cell captures an antigen during activation of NKT cells, the dendritic cell becomes mature, and can induce an antigen specific T cell.
Non-patent reference 4 describes that, in maturation of dendritic cells by NKT cells, IFN-γ and TNF-α, as well as CD40 signal are important.
Non-patent reference 5 describes that human NKT cells activated in vitro can kill CD1d expression target presenting α-GalCer.
Non-patent reference 6 describes that CD1d is expressed on B cell leukemia cell line, and can mediate presentation of α-GalCer to NKT cells.
Non-patent reference 7 describes that, in activation of NKT cells, antigen specific T cells cannot be induced without maturation of dendritic cells and antigen uptake by the cells.
Non-patent reference 8 describes that administration of antigen during activation of NKT cells by the administration of α-GalCer induces an antigen specific immune response.
Non-patent reference 9 is concerned with the use of irradiated tumor cells for immunotherapy.

However, a method enabling simultaneous induction of NKT cell activation and T-cell immune response to tumors, which is earnestly desired for the establishment of an immunotherapy of tumors, has not been developed as yet.
non-patent reference 1: Fujii et al., Nature Immunology 3: 867-874 (2002)
non-patent reference 2: Fujii et al., Journal of Immunological Methods 272: 147-159 (2003)
non-patent reference 3: Fujii et al., The Journal of Experimental Medicine 198: 267-279 (2003)
non-patent reference 4: Fujii et al., The Journal of Experimental Medicine 199: 1607-18 (2004)
non-patent reference 5: Metelitsa et al., The Journal of Immunology 167: 3114-3122 (2001)
non-patent reference 6: Fais et al., International Journal of Cancer 109: 402-11 (2004)
non-patent reference 7: Hermans et al., The Journal of Immunology 171: 5140-5147 (2003)
non-patent reference 8: Silk et al., The Journal of Clinical Investigation 114, 1800-11 (2004)
non-patent reference 9: Liu et al., Journal of Experimental Medicine, 202: 1507-1516 (2005)

### Disclosure of the Invention

### Problems to be Solved by the Invention

Accordingly, an object of the present invention is to provide an immunoinducer which simultaneously induces NKT cell activation and T-cell immune response to tumors.

### Means of Solving the Problems

The present inventors unexpectedly and surprisingly found that a tumor cell pulsed with a CD1d ligand induces a very strong immune response specific to the tumor cell, particularly simultaneous activation of NK/NKT cells and T-cell immune response. The present inventors have further conducted intensive studies and found that the above-mentioned antitumor immune response is dependent on CD1d expressed in tumor cell, and the like. Based on the above findings, the present inventors have concluded that a tumor antigen and CD1d co-expressing cell, which is pulsed with a CD1d ligand, can induce a very strong immune response specific to the tumor cell, particularly, simultaneous activation of NK/NKT cells and T-cell immune response. Such methodology is considered widely applicable not only to immunotherapy of tumor but also immunotherapy of cells and organisms expressing a specific target antigen, for example, virus-infected cells and pathogens (e.g., virus). An idea of using a target antigen and CD1d co-expressing cell, which is pulsed with a CD1d ligand, for the induction of immunity to the target antigen (including product containing the target antigen) is not described or suggested in the above-mentioned prior art references.

The present inventors have completed the present invention based on the above.

That is, the present invention provides the following invention.
[1] A method of preparing an immunoinducer to a target antigen, comprising pulsing a target antigen and CD1d co-expressing cell with a CD1d ligand thereby presenting the CD1d ligand on the surface of the cell via CD1d, wherein the immunoinducer is for use in a method of prophylaxis or treatment of a neoplastic disease or infection and wherein the co-expressing cell is produced by:
   (i) transforming a cell with a vector that expresses at least a target antigen or CD1d; or
   (ii) introducing the target antigen and CD1d, such that the target antigen and CD1d will be co-expressed in the object cell, or expression of the target antigen and/or CD1d will be enhanced in the target antigen and CD1d co-expressing cell, provided that the cell is not a dendritic cell.
[2] An immunoinducer to a target antigen, comprising a target antigen and CD1d co-expressing cell, wherein the co-expressing cell has been pulsed with a CD1d ligand and the CD1d ligand is thereby presented on the surface of the cell via CD1d and wherein the co-expressing cell is produced by:
   (i) transforming a cell with a vector that expresses at least a target antigen or CD1d; or
   (ii) introducing the target antigen and CD1d, such that the target antigen and CD1d will be co-expressed in the object cell, or expression of the target antigen and/or CD1d will be enhanced in the target antigen and CD1d co-expressing cell, wherein the immunoinducer is for use in a method of prophylaxis or treatment of a neoplastic disease or infection, provided that the cell is not a dendritic cell.
[3] A composition comprising the immunoinducer of [2], and an adjuvant.
[4] Use of a target antigen and CD1d co-expressing cell capable of activating immunity to the target antigen, wherein the co-expressing cell has been pulsed with a CD1d ligand and the CD1d ligand is thereby presented on the surface of the cell via CD1d, in the manufacture of a medicament for use in a method of prophylaxis or treatment of a neoplastic disease or infection, provided that the cell is not a dendritic cell, wherein the co-expressing cell is produced by:
   (i) transforming a cell with a vector that expresses at least a target antigen or CD1d; or
   (ii) introducing the target antigen and CD1d, such that the target antigen and CD1d will be co-expressed in the object cell, or expression of the target antigen and/or CD1d will be enhanced in the target antigen and CD1d co-expressing cell.
[5] The use of [4], wherein the medicament comprises an adjuvant.

### Brief Description of the Drawings

Fig. 1 shows the IFN-γ production amount of a mouse liver-derived mononuclear cell co-cultured with a tumor cell pulsed with α-GalCer. CD1dB16: CD1d expression enhanced B16 CD1dEL4: CD1d expression enhanced EL4 DC: dendritic cell WT:wild-type JαKO:Ja281gene deficient mouse (Va14⁺NKT cell deficient mouse) -: not pulsed with α-GalCer +: pulsed with α-GalCer
Fig. 2 shows IFN-γ production amount in mouse immunized with a tumor cell pulsed with various concentrations of α-GalCer. Gal: α-GalCer DC/G: dendritic cell pulsed with α-GalCer B16/G: B16 pulsed with α-GalCer CD1dB16/G: CD1d expression enhanced B16 pulsed with α-GalCer
Fig. 3 shows difference in antitumor immunity in lung metastasis models.
Fig. 4 shows antitumor immune response in mouse immunized with a tumor cell pulsed with α-GalCer.
Fig. 5 shows relative expression levels of CD1d mRNA among tumor cell lines, as measured by a real time RT-PCR method.
Fig. 6 shows relative expression levels of CD1d protein among tumor cell lines, as measured by flow cytometry. DC: dendritic cell CD1dB16: CD1d expression enhanced B16 CD1dEL4: CD1d expression enhanced EL4 isotype:ratIgG2b
Fig. 7 shows a killing effect provided by a liver-derived mononuclear cell on tumor cells pulsed or not pulsed with CD1d ligand. E/T ratio: effecter (E)/target (T) ratio, where liver-derived mononuclear cell containing NKT cell and NK cell corresponds to E, and a tumor cell corresponds to T.
Fig. 8 shows the measurement results of CFSE uptake by dendritic cell using a flow cytometer.

### Best Mode for Carrying out the Invention

### (1. Cells)

The present invention provides an immunoinducer to a target antigen, comprising a target antigen and CD1d co-expressing cell, wherein the co-expressing cell has been pulsed with a CD1d ligand and the CD1d ligand is thereby presented on the surface of the cell via CD1d and wherein the co-expressing cell is produced by:
(i) transforming a cell with a vector that expresses at least a target antigen or CD1d; or
(ii) introducing the target antigen and CD1d, such that the target antigen and CD1d will be co-expressed in the object cell, or expression of the target antigen and/or CD1d will be enhanced in the target antigen and CD1d co-expressing cell, wherein the immunoinducer is for use in a method of prophylaxis or treatment of a neoplastic disease or infection, provided that the cell is not a dendritic cell. Such co-expressing cell pulsed with a CD1d ligand has an ability to activate the immunity to the target antigen.

CD1d ligand refers to a substance presented on a CD1d expression antigen presenting cell (APC), which can activate NKT cells. Examples of "CD1d ligand" include α-GalCer (α-galactosylceramide), α-C-GalCer ((α-C-galactosylceramide), iGB3 (isoglobotrihexosylceramide), GD3 (ganglioside 3), GSL-1 (α-linked glucuronic acid), GSL-1'SA (galacturonic acid), with preference given to α-GalCer and α-C-GalCer. The cell of the present invention presents CD1d ligand on the cell surface via CD1d, and can activate NKT cells.

The present inventors first found that a predetermined cell (e.g., a target antigen and CD1d co-expressing cell) pulsed with a CD1d ligand is highly useful for immunotherapy. For example, as the target antigen and CD1d co-expressing cell, cells including mouse-derived melanoma cell line B16 and mouse-derived T lymphoma cell line EL4 are already present. Without such finding by the present inventors, however, there is no motivation to intentionally produce a target antigen and CD1d co-expressing cell that can be used for the method developed by the present inventors.

In the present specification, when a target antigen and CD1d co-expressing cell pulsed with a CD1d ligand is particularly intended, it may be as abbreviated as, where necessary, "pulsed cell of the present invention" (which means a cell that may have an ability to activate the immunity to the target antigen). "Non-pulsed cell" means a cell that can acquire an ability to activate immunity to the target antigen after being pulsed with a CD1d ligand, but cannot have an ability to activate immunity to the target antigen since it is not pulsed with CD1d ligand.

The cell of the present invention may be isolated and/or purified. Isolation and purification can be performed by a method known per se.

The cell of the present invention may also be derived from any animal species. Examples of such animal species include mammals such as human, monkey, chimpanzee, dog, cat, horse, bovine, swine, sheep, goat, mouse, rat, guinea pig, hamster, rabbit and the like, with preference given to a cell derived from human from the aspect of clinical application.

The cell of the present invention may be a cell type derived from any tissue. Examples of such tissue include stomach, small intestine (e.g., duodenum, jejunum, ileum, colon), large intestine, rectal, lung, pancreas, kidney, liver, thymus, spleen, thyroid gland, adrenal gland, prostate, ovary, uterus, bone marrow, skin, and peripheral blood. The cell of the present invention may also be a particular cell type in the above-mentioned tissues or a cell type in a tissue other than the above-mentioned tissues. Examples of such cell type include epithelial cell, endothelial cell, epidermal cell, interstitial cell, fibroblast, adipocyte, mammary cell, mesangial cell, pancreatic β cells, nerve cell, glial cell, immune cell (e.g., T cells, B cells, NK cell, NKT cell, macrophage, mast cell, neutrophil, basophil, eosinophils, monocyte), and precursor cells and stem cells of these cells.

Furthermore, the cell of the present invention may be a cell obtained from an animal (e.g., primary cultured cell) or a cell line. The cell line may be an existing cell line or a newly prepared cell line. The cell line can be produced by a method known per se.

More importantly, the cell of the present invention is a cell that expresses both a target antigen and CD1d.

A target antigen is an antigen expressed on an abnormal cell or pathogen, and is not particularly limited as long as intracorporeal disappearance of the abnormal cell or pathogen, or a decrease in the amount of the abnormal cell or pathogen is expected due to an immune action targeting the antigen. Examples of the target antigen include tumor antigen and pathogenic antigen. The cell of the present invention can express one or more target antigens to the same target.

The tumor antigen may be an antigen of a solid tumor including epithelial and nonepithelial tumors, or a tumor in a hematopoietic tissue. While the solid tumor antigen is not particularly limited, for example, MART-1/Melan-A, Mage-1, Mage-3, gp100, tyrosinase, CEA, PSA, CA-125, erb-2, Muc-1, Muc-2, TAG-72, AES, FBP, C-lectin, NY-ESO-1, galectin-4/NY-CO-27, Pec60, HER-2/erbB-2/neu, telomerase, G250, Hsp105, point mutation ras oncogene, point mutation p53 oncogene and carcinoembryonic antigen can be mentioned (e.g., JP-A-2005-139118, JP-A-2004-147649, JP-A-2002-112780, JP-A-2004-222726). While the tumor (e.g., leukemia) antigen in a hematopoietic tissue is not particularly limited, for example, proteinase 3, WT-1, hTERT, PRAME, PML/RAR-a, DEK/CAN, cyclophilin B, TEL-MAL1, BCR-ABL, OFA-iLRP, Survivin, idiotype, Sperm protein 17, SPAN-Xb, CT-27 and MUC1 can be mentioned.

The pathogenic antigen may be a pathogenic virus antigen, a pathogenic microorganism antigen, or a pathogenic protozoan antigen. While a pathogenic virus antigen is not particularly limited, for example, GP-120, p17, GP-160 (which are HIVs), NP, HA (which are influenza viruses), HBs Ag, HBV envelope protein, core protein, polymerase protein, NS3, NS5 (which are hepatitis viruses), HSVdD (simple herpes virus), EBNA1, 2, 3A, 3B and 3C, LMP1 and 2, BZLF1, BMLF1, BMRF1, BHRF1 (which are EB viruses), Tax (HTLV-I), SARS-CoV spike protein (SARS virus), CMV pp5, IE-1 (which are CMVs), E6, E7 proteins (which are HPVs) can be mentioned (e.g., JP-A-2004-222726). Examples of the pathogenic microorganism antigen include antigens expressed in pathogenic bacterium (e.g., chlamydiae, mycobacteria, Legionella) or pathogenic yeast (e.g., aspergillus, Candida). Examples of the pathogenic protozoan antigen include antigens expressed in malaria or schistosomemalaria.

CD1d is known to be a major histocompatible antigen (MHC)-like molecule that presents glycolipid rather than peptide. CD1d is expressed in antigen presenting cell (e.g., dendritic cell), epithelial cells in tissues in the intestine, liver and the like, some tumor cells (e.g., solid tumor cell, leukemia cell) and virus-infected cells.

The cell of the present invention may also be a normal cell or abnormal cell. The normal cell refers to a cell not in a pathogenic state. On the other hand, the abnormal cell refers to a cell in a pathogenic state. Examples of the abnormal cell include tumor cell and virus-infected cell. The tumor cell may be one corresponding to the aforementioned cell type. Examples of the virus-infected cell include the aforementioned cell type infected with a pathogenic virus. While the pathogenic virus is not particularly limited, for example, human immunodeficiency virus (HIV), hepatitis virus (e.g., type A, type B, type C, type D and type E hepatitis virus), influenza virus, simple herpes virus, West Nile fever virus, human papillomavirus, equine encephalitis virus and human T-cell leukemia virus (e.g., HTLV-I) can be mentioned.

The cell of the present invention may also be non-transformant or transformant. When used in the present specification, the transformation refers to an artificial transgenic manipulation, and the transformant means a cell produced by such artificial manipulation. Therefore, a cell produced by a non-artificial manipulation is treated as one not falling under a transformant in the present specification. To be more precise, when the cell of the present invention is a transformant, the cell of the present invention can be produced by transforming, as a host cell, a cell that does not express both a target antigen and CD1d, or a cell that naturally expresses one or both of the target antigen and CD1d, with a vector that expresses at least a target antigen, or a vector that expresses at least CD1d or 1 or 2 vectors that express at least a target antigen and CD1d (detail of the expression vector is described later). In transformation, the host cell may be any cell, and can be, for example, a normal cell or an abnormal cell (e.g., tumor cell such as leukemia cell and the like, virus-infected cell), or a cell non-naturally expressing a target antigen and CD1d, a cell naturally expressing a target antigen, a cell naturally expressing CD1d, or a cell naturally expressing a target antigen and CD1d.

The cell of the present invention may also be a target antigen-introduced cell or a target antigen-unintroduced cell. When used in the present specification, introduction of a target antigen means an artificial target antigen transfer operation, and an introduced cell of a target antigen means a cell produced by such an artificial operation. Therefore, a cell produced by a non-artificial operation is treated as one not falling under an introduced cell of a target antigen in the present specification. To be more precise, when the cell of the present invention is an introduced cell of a target antigen, the cell of the present invention may be produced by incorporating a target antigen into, as a host cell, a cell that does not express both the target antigen and CD1d, or a cell that naturally expresses both or one of the target antigen and CD1d.

The cell of the present invention may also be a cell derived from an antigen presenting cell (APC) or non-APC, each being naturally-occurring. Examples of the naturally-occurring APC include, macrophage, B cell, Langerhans cell and activated T cell. Hence, the cell of the present invention may be a naturally-occurring APC or non-APC harboring an incorporated target antigen or transformed with a vector that expresses at least a target antigen, a vector that expresses at least CD1d, or 1 or 2 vectors that express at least a target antigen and CD1d.

The cell of the present invention may also be a cell derived from an antigen presenting cell expressing CD1d (CD1d-expressing APC) or non-CDld-expressing APC, each being naturally-occurring. The CD1d-expressing APC refers to a cell capable of activating NKT cells, which has CD1d on the cell surface thereof. Examples of the CD1d-expressing APC include macrophage and B cell. Hence, the cell of the present invention may be a naturally-occurring CD1d expressing APC or non-CDld-expressing APC harboring an incorporated target antigen or transformed with a vector that expresses at least a target antigen, a vector that expresses at least CD1d, or 1 or 2 vectors that express at least a target antigen and CD1d.

As the cell of the present invention, moreover, a cell derived from an individual to be immunized with a cell (syngeneic to subject of administration), a cell derived from another individual allogeneic to an individual to be immunized with a cell (cell allogeneic to subject of administration), or a cell derived from an individual heterogeneous to an individual to be immunized with a cell (heterogeneous to subject of administration) can be used. Since the object is induction of T-cell immune response to a tumor antigen of oneself, a syngeneic cell is preferable.

The pulsed cell of the present invention is, as mentioned below, useful as a pharmaceutical agent, an immunity activator and the like. The non-pulsed cell of the present disclosure is useful, for example, for the production of the pulsed cell of the present invention.

### (2. Preparation and identification methods)

The present invention provides a method for preparing the cell of the present invention.

In one embodiment, the preparation method of the present invention comprises a method for preparing the non-pulsed cell of the present disclosure. The preparation method of the non-pulsed cell of the present disclosure may include treating a cell such that the target antigen and CD1d will be co-expressed in the object cell, or expression of the target antigen and/or CD1d will be enhanced in the target antigen and CD1d co-expressing cell.

For example, when the preparation method of the present invention includes treatment of a cell such that the target antigen and CD1d will be co-expressed in the object cell, the object cell may be a cell that does not express both the target antigen and CD1d, or a cell that expresses only one of the target antigen and CD1d.

In addition, when the preparation method of the present invention includes treatment of a cell such that the expression of the target antigen and/or CD1d will be enhanced in the target antigen and CD1d co-expressing cell, the expression of the target antigen and/or CD1d can be enhanced to the degree that the treatment effect of the immunotherapy using the cell of the present invention is sufficiently increased.

The treatment in the preparation method of the present invention may be transformation or an operation to introduce a target antigen. To be more precise, the preparation method of the present invention may include (a) transforming a CD1d-expressing cell with a vector that expresses at least a target antigen, (b) transforming a target antigen-expressing cell with a vector that expresses at least CD1d, (c) transforming a cell with 1 or 2 vectors that express at least a target antigen and CD1d, (d) incorporating a target antigen itself into a CD1d-expressing cell, or (e) transforming a cell with a vector that expresses at least CD1d, and incorporating the target antigen itself into the cell, or (f) incorporating a target antigen itself into a cell, and transforming the cell with a vector that expresses at least CD1d. The transformation of the cell can be performed by a method known per se such as a lipofection method, a calcium phosphate precipitation method, an electroporation method and the like. The introduction of the target antigen into the cell can be performed, for example, using hyperosmotic shock, electroporation, liposome or a fusion protein of a signal peptide, which enables intracellular uptake, and a target antigen.

In the aforementioned preparation method, the CD1d-expressing cells in (a) and (d) may be a cell that expresses CD1d and does not express a target antigen, or a cell that expresses both a target antigen and CD1d. The target antigen-expressing cell in (b) may be a cell that expresses a target antigen and does not express CD1d, or a cell that expresses both a target antigen and CD1d. The cells in (c), (e) and (f) may be a cell that does not express both a target antigen and CD1d, a cell that expresses a target antigen and does not express CD1d, a cell that expresses CD1d and does not express a target antigen, or a cell that expresses both a target antigen and CD1d.

The preparation method of the present invention is a preparation method of the pulsed cell of the present invention. The preparation method of the pulsed cell of the present invention may include treating a target antigen and CD1d co-expressing cell with a CD1d ligand in a culture medium. By such a treatment, the CD1d ligand is presented on the target antigen and CD1d co-expressing cell, and the co-expressing cell can acquire an ability to activate immunity to the target antigen.

The culture medium can be prepared using, as a basal medium, a medium used for culturing animal cells. Examples of the basal medium include MEM medium, DMEM medium, αMEM medium, Ham's medium, RPMI1640 medium, Fischer's medium, and a mixed medium thereof. The culture medium can contain, for example, serum (e.g., FCS), serum replacement (e.g., knockout Serum Replacement (KSR)), fatty acid or lipid, amino acid, vitamin, growth factor, cytokine, antioxidant, 2-mercaptoethanol, pyruvic acid, buffering agent, inorganic salts and the like. Other culture conditions such as culture temperature, CO₂ concentration and the like can be set as appropriate. While the culture temperature is not particularly limited, for example, it is about 30 - 40°C, preferably about 37°C. The CO₂ concentration is, for example, about 1 - 10%, preferably about 5%. Other conditions such as the number of cells to be cultured, concentrations of various factors and the like can be appropriately set by methods known per se.

The cell to be used for the preparation of the pulsed cell of the present invention is not particularly limited as long as it is a target antigen and CD1d co-expressing cell produced by:
(i) transforming a cell with a vector that expresses at least a target antigen or CD1d; or
(ii) introducing the target antigen and CD1d, such that the target antigen and CD1d will be co-expressed in the object cell, or expression of the target antigen and/or CD1d will be enhanced in the cell, and the cell is not a dendritic cell.

When a treated cell (e.g., transformant) is used as a target antigen and CD1d co-expressing cell, the preparation method of the present invention may include treatment of a cell such that the target antigen and CD1d will be co-expressed in the object cell or the expression of the target antigen and/or CD1d will be enhanced in the target antigen and CD1d co-expressing cell, and obtaining the target antigen and CD1d co-expressing cell. The methodology is the same as for the aforementioned preparation method of the present invention. The object cell may be a cell derived from an individual to be administered with the cell of the present invention (syngeneic cell), a cell derived from an individual allogeneic to an individual to be administered with the cell of the present invention (allogeneic cell), or a cell derived from an individual heterogeneous to an individual to be administered with the cell of the present invention (heterogeneous cell).

On the other hand, when an untreated cell (e.g., non-transformant) is used as a target antigen and CD1d co-expressing cell, the preparation method of the present disclosure may further include recovering the target antigen and/or CD1d co-expressing cell from a biological sample obtained from an individual to give the target antigen and CD1d co-expressing cell. Examples of the biological sample obtained from an individual include biological samples such as tumor mass, peripheral blood, liver, lymph node, spleen and the like. Examples of the cell obtained from an individual include abnormal cells such as tumor cells (e.g., leukemia cell), virus-infected cell and the like (the abnormal cell may be of the same type as the cell of the present invention). The individual may be of the same type as the aforementioned animal species. The individual may also be the same as or different from the individual to be administered with the cell of the present invention.

When an untreated cell (cell itself obtained from an individual) is used as a target antigen and CD1d co-expressing cell, it is possible to identify where necessary using a part of the biological sample containing the non-transformant whether or not the cell contained in the sample expresses both a target antigen and CD1d. The present disclosure also provides such identification method. The present inventors first found that a target antigen and CD1d co-expressing cell pulsed with a CD1d ligand is highly useful for an immunotherapy. Without such finding by the present inventors, there is no motivation to intentionally identify a target antigen and CD1d co-expressing cell (particularly, a co-expressing cell obtained from an individual) that can be used for the method developed by the present inventors. When a tumor is obtained as a biological sample, the tumor antigen is considered to be naturally expressed in a cell contained in the sample. In such a case, therefore, it is not always necessary to confirm expression of the tumor antigen in the identification method of the present disclosure, and confirmation only of the CD1d expression suffices. The same applies to other target antigens.

Therefore, the identification method of the present disclosure may include measurement of expression of CD1d or expressions of target antigen and CD1d in the object cell. Examples of the object cell include treated cell and untreated cell, and existing cell and novel cell. Examples of the object cell and/or a cell obtained from an individual include abnormal cells such as tumor cell, virus-infected cell and the like (the abnormal cell may be of the same type as the cell of the present invention).

The expression of the target antigen and CD1d can be measured by a method known per se and using, for example, plural primers (e.g., primer pair(s)) that can amplify target antigen and/or CD1d, nucleic acid probe that can detect target antigen and/or CD1d, or an antibody to target antigen and/or CD1d.

The preparation method of the present invention is useful, for example, for the preparation of the cell of the present invention having the above-mentioned various advantages. The identification method of the present disclosure is useful, for example, for screening for a cell necessary for preparation of the pulsed cell of the present invention.

### (3. Expression vector)

When used in the present specification, a vector that expresses at least a target antigen means a vector that expresses a target antigen alone, or a vector that expresses a target antigen and other useful factors. Examples of the vector that expresses at least a target antigen include a target antigen expression vector and a target antigen and CD1d co-expression vector.

The vector that expresses at least CD1d means a vector that expresses a vector that expresses CD1d alone, and a vector that expresses CD1d and other useful factors. Examples of the vector that expresses at least CD1d include a CD1d expression vector and a target antigen and CD1d co-expression vector.

The 1 or 2 vectors that express at least a target antigen and CD1d means a single vector that expresses both a target antigen and CD1d, or a combination of a vector that expresses at least a target antigen and a vector that expresses at least CD1d. Examples of the 1 or 2 vectors that express at least a target antigen and CD1d include a target antigen and CD1d co-expression vector and a combination of a target antigen expression vector and a CD1d expression vector.

The present disclosure also provides such a co-expression vector.

The co-expression vector of the present disclosure may contain a first polynucleotide encoding a target antigen and a second polynucleotide encoding CD1d. The co-expression vector of the present disclosure may also contain a promoter functionally linked to the above-mentioned first and the second polynucleotides. The functional linkage of the promoter means that the promoter is bound to the polynucleotide in such a manner as to permit expression of a factor encoded by the polynucleotide under regulation.

To be more precise, the co-expression vector of the present disclosure may be a polycistronic mRNA expression vector. The polycistronic mRNA expression vector may contain a first polynucleotide and second polynucleotide conjugate, which enables expression of a target antigen and CD1d polycistronic mRNA, and a promoter functionally linked to the conjugate.

The co-expression vector of the present disclosure may also be a non-polycistronic mRNA expression vector. The non-polycistronic mRNA expression vector may contain a first polynucleotide and a first promoter functionally linked to the polynucleotide, as well as a second polynucleotide and a second promoter functionally linked to the polynucleotide.

The promoter to be used for a co-expression vector is not particularly limited as long as it can function in a cell to be introduced into. Examples thereof include virus promoters such as SV40 derived early promoter, cytomegalovirus LTR, Rous sarcoma virus LTR, MoMuLV derived LTR, adenovirus derived early promoter and the like, promoters of mammalian constituent protein gene such as β-actin gene promoter, PGK gene promoter, transferrin gene promoter and the like, and the like can be mentioned. In addition, a promoter specific to a cell to be introduced into may also be used. Examples of such promoter include abnormal cell specific promoters such as tumor cell specific promoter, virus-infected cell specific promoter (e.g., virus late promoter) and the like. Not only the co-expression vector of the present disclosure but also the expression vector to be used in the present invention can have such a promoter.

The co-expression vector preferably contains a transcription termination signal, i.e., terminator region, at the downstream of oligo(poly)nucleotide encoding a nucleic acid molecule. It may further contain a selection marker gene (gene imparting resistance to pharmaceutical agents (e.g., tetracycline, ampicillin, kanamycin, hygromycin, phosphinothricin), gene complementing auxotrophic mutation etc.) for selecting a transformed cell.

A vector to be the basic skeleton of a co-expression vector may be, for example, a plasmid or a virus vector (e.g., vector derived from virus such as adenovirus, retrovirus, adeno-associated virus, herpesvirus, vaccinia virus, poxvirus, polio virus, sindbis virus, Hemagglutinating Virus of Japan, lentivirus and the like).

The co-expression vector of the present disclosure is, like the aforementioned agent of the present invention, useful, for example, as a pharmaceutical agent, and for the activation of immune cells and preparation of the cell of the present invention.

### (4. Agent and composition)

The present invention provides an agent (or composition) containing the cell of the present invention, for use in a method of prophylaxis or treatment of a neoplastic disease or infection.

The animal species to which the agent of the present invention is administered may be the same as the animal species from which the target antigen and CD1d co-expressing cell derives. Thus, the agent of the present invention can achieve immunization with a syngeneic, allogeneic or heterogeneous cell in the immunization with the cell of the present invention.

The agent of the present invention may contain, in addition to the cell of the present invention pulsed with a CD1d ligand, any carrier, for example, pharmaceutically acceptable carriers and/or adjuvant. Examples of the pharmaceutically acceptable carrier include, but are not limited to, diluents such as water, saline and the like, and the like. While the adjuvant is not particularly limited as long as it can enhance the antigenicity of the target antigen, for example, BCG, trehalose dimycolate (TDM), Merck65, AS-2, aluminum phosphate, aluminum hydroxide, keyhole limpet hemocyanin, dinitrophenol, dextran and TLR ligand (e.g., lipopolysaccharide (LPS), CpG) can be mentioned.

The agent of the present invention is useful, for example, as a pharmaceutical agent or reagent. To be more precise, the agent of the present invention is useful for the prophylaxis or treatment of neoplastic diseases or infections, or immunotherapy (e.g., activation of immunocytes such as NK/NKT cells, T cells and the like). Examples of the neoplastic diseases possibly prevented or treated by the agent of the present invention include tumors in the aforementioned tissues and cell types, such as solid tumor (e.g., epithelial tumor, non-epithelial tumor), and tumors in hematopoietic tissues. To be more precise, examples of the solid tumor possibly prevented or treated by the agent of the present invention include digestive cancer (e.g., gastric cancer, colon cancer, colorectal cancer, rectal cancer), lung cancer (e.g., small cell cancer, non-small cell cancer), pancreatic cancer, kidney cancer, liver cancer, thymus cancer, spleen cancer, thyroid cancer, adrenal gland cancer, prostate cancer, urinary bladder cancer, ovarian cancer, uterine cancer (e.g., endometrial carcinoma, cervical cancer), bone cancer, skin cancer, sarcoma (e.g., Kaposi's sarcoma), melanoma, blastoma (e.g., neuroblastoma), adenocarcinoma, carcinoma planocellulare, non-carcinoma planocellulare, brain tumor, as well as recurrence and metastasis of these solid tumors. Examples of the tumor in the hematopoietic tissue possibly prevented or treated by the agent of the present invention include leukemia (e.g., acute myeloid leukemia (AML), chronic myelocytic leukemia (CML), acute lymphocytic leukemia (ALL), chronic lymphocytic leukemia (CLL), adult T cell leukemia (ATL), myelodysplastic syndrome (MDS)), lymphoma (e.g., T lymphoma, B lymphoma, Hodgkin's lymphoma), myeloma (multiple myeloma), as well as recurrence of these tumors. Examples of the infections possibly treated by the agent of the present invention include infections caused by the aforementioned pathogens.

The present inventors have also found at this time that a target antigen and CD1d co-expressing cell pulsed with a CD1d ligand can have an ability to simultaneously induce activation of NK/NKT cells and T-cell immune response. It is known that NK/NKT cells have MHC class I-non-expressing cell as a good target, and T cells have MHC class I-expressing cell as a good target. Accordingly, the agent of the present invention is advantageous in that an effect on various target antigen-expressing cells can be expected.

While the dose of the agent of the present invention varies depending on the kind of the pulsed cell of the present invention (pulsed with a CD1d ligand), expression levels of the target antigen and CD1d in the pulsed cell of the present invention, administration mode, severity of disease, animal species of the subject of administration, and acceptability, body weight, age and the like of the subject of administration and cannot be generalized, cells in a number that affords a desired immunoactivity can be appropriately administered.

The present invention is explained in more detail in the following by referring to Examples, which are mere exemplifications and do not limit the scope of the present invention.

### Examples

### (Materials and method)

### (Preparation of tumor cell pulsed with α-GalCer)

As the tumor cell line pulsed with α-GalCer, mouse-derived melanoma cell line B16 and mouse-derived T lymphoma cell line EL4 were used. α-GalCer was added to B16 (2x10⁴ cells/ml) or EL4 (1x10⁵ cells/ml) to a concentration of 500 ng/ml, and the cells were cultured at 5% CO₂, 37°C. As the culture medium, 10% FCS-containing RPMI (10 ml) was used. After culturing for 2 days, B16 or EL4 pulsed with α-GalCer was washed 4 times with PBS, and then collected.

### (Preparation of dendritic cell pulsed with α-GalCer)

Bone marrow cells were collected from the femur and shin bone of wild-type mice (C57BL/6, 6- to 8-week-old, female), and CD4, CD8, B220 or I-Ab positive cells were removed using antibodies and complements. The obtained cells were adjusted to 1x10⁶ cells/ml with GM-CSF (10 ng/ml) and 5% FCS-containing RPMI, and cultured in a 24 well plate. The medium was changed every two days, and α-GalCer was added to 100 ng/ml on day 6. On day 7, LPS (100 ng/ml) was further added, and the dendritic cells were matured. The cells were collected on day 8, and washed with PBS.

### (Preparation of mononuclear cell)

The mononuclear cells were prepared from the spleen and liver. Spleen was removed from wild-type mice (C57BL/6, 6- to 8-week-old, female), filtered with a cell strainer, red blood cells were hemolyzed with ACK lysing buffer, and the mononuclear cells were washed to give spleen-derived mononuclear cells. In addition, the liver was removed from wild-type mice or Jα281 gene deficient mice (Vα14⁺NKT cell deficient mouse: e.g., see Fujii et al., The Journal of Experimental Medicine 198: 267-279 (2003), and Fujii et al., The Journal of Experimental Medicine 199: 1607-18 (2004)), filtered with a stainless mesh, and a mononuclear cell layer was separated using a percoll by the density gradient centrifugation method to give liver-derived mononuclear cells.

### (Production of lung metastatic animal model and evaluation thereof)

B16 melanoma cells were adjusted to 5x10⁵/200 µl with PBS, and intravenously administered to mice (C57BL/6, 6- to 8-week-old, female) from the tail vein. After 2 weeks, the lung was removed from the mice and the number of metastatic B16 cells in the lung was measured.

### Example 1: In vitro activation of NKT/NK cell by tumor cell pulsed with CD1d ligand

B16 or EL4 pulsed with α-GalCer, or dendritic cell (control) pulsed with α-GalCer was co-cultured with a mononuclear cell (fraction containing NKT/NK cell) derived from the liver of a wild-type mouse or Ja281 gene deficient mouse (Va14⁺NKT cell deficient mouse), and the level of IFN-γ in the culture supernatant was measured by ELISA and using an anti-IFN-y antibody. Since IFN-γ is a substance specifically produced by activated NKT/NK cells, the level of IFN-γ in the culture supernatant can be an index of activated NKT/NK cells. In addition, an experiment similar to the above was performed using B16 and EL4 into which a CD1d expression retrovirus vector (produced by using mouse CD1d gene (GenBank Accession No.: NM-007639) had been introduced).

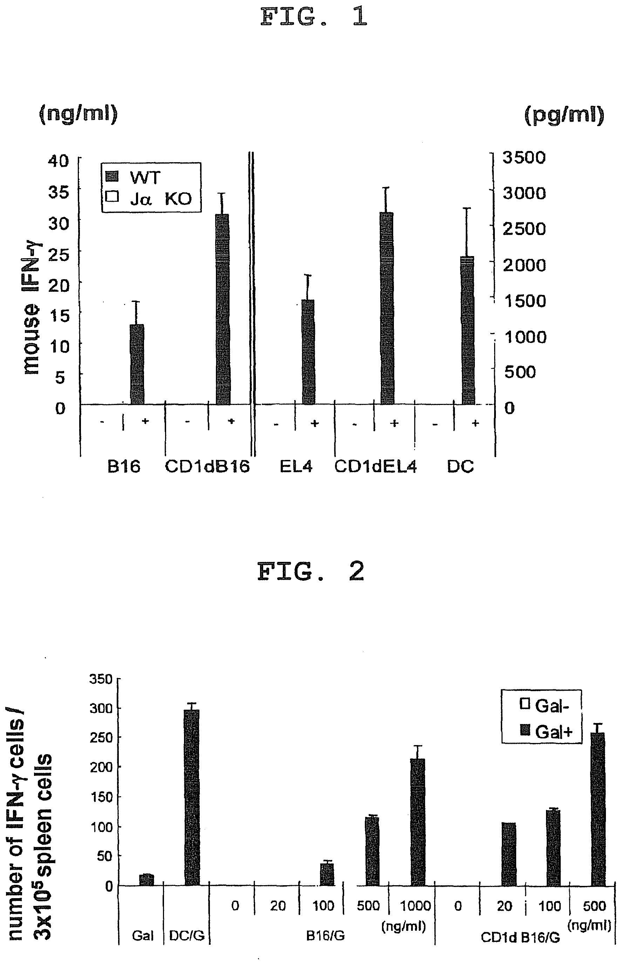

As a result, the both of B16 and EL4 pulsed with α-GalCer activated NKT/NK cells (Fig. 1). In addition, B16 and EL4 pulsed with α-GalCer and containing a CD1d expression retrovirus vector activated NKT/NK cells more than B16 and EL4 pulsed with α-GalCer and free of the vector (Fig. 1).

From the foregoing, it has been clarified that tumor cells pulsed with a CD1d ligand activate NKT/NK cells in vitro in a CD1d expression level dependent manner.

### Example 2: In vivo activation of NKT cells by tumor cells pulsed with CD1d ligand

B16 (5x10⁵ cells) or dendritic cells (control, 1x10⁶ cells) pulsed with α-GalCer were intravenously administered to mice (C57BL/6, 6- to 8-week-old, female) from the tail vein. Two days after the administration, the spleen was isolated from the mice, filtered with a cell strainer, red blood cells were hemolyzed with ACK lysing buffer, and the splenocytes were adjusted with 5% FCS-containing RPMI. (3x10⁵ cells/well) was cultured for 16 hr in the presence of α-GalCer in the same manner as in Example 1 and the level of IFN-γ in the culture supernatant was measured by ELISPOT and using an anti-IFN-y antibody. In addition, an experiment similar to the above was performed using B16 into which a CD1d expression retrovirus vector had been introduced.

As a result, the dendritic cells pulsed with α-GalCer induced α-GalCer reactive IFN-γ producing NKT cells (Fig. 1). When B16 was used, the efficiency decreased but α-GalCer reactive IFN-γ producing NKT cells could be induced in the same manner, where the induction capability was dependent on the concentration of α-GalCer used for pulsing and the induction capability was more efficient in B16 that strongly expresses CD1d (Fig. 2).

From the foregoing, it has been clarified that tumor cells pulsed with a CD1d ligand activate NKT cells in vivo in an α-GalCer concentration dependent manner.

### Example 3: Antitumor effect on lung metastatic animal model

The antitumor effect of IFN-γ production by tumor cells pulsed with a CD1d ligand was examined. B16 or CD1d expression enhanced B16, or B16 pulsed with α-GalCer or CD1d expression enhanced B16 pulsed with α-GalCer (both at 5x10⁵ cells) was intravenously administered to mice. Then, after 14 days from the administration, the lung was removed from the mice, and the antitumor effect was evaluated.

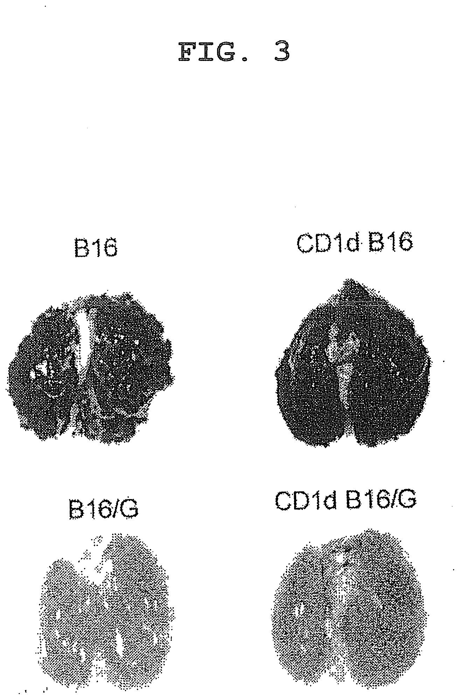

As a result, the metastatic model mice administered with B16 or CD1d expression enhanced B16 showed a tumor increase, but the tumor apparently disappeared in the model mice administered with B16 pulsed with α-GalCer or CD1d expression enhanced B16 pulsed with α-GalCer (Fig. 3).

From the foregoing, it has been clarified that tumor cells pulsed with a CD1d ligand show an antitumor effect by an enhanced spontaneous immune response.

### Example 4: Induction of cytotoxic T lymphocyte to tumor antigen

B16 (5×10⁵ cell) was subcutaneously administered to the mouse (wild-type mouse) administered with CD1d expression enhanced B16 pulsed with α-GalCer, whose tumor was confirmed to have disappeared in Example 3. In addition, B16 (1×10⁵ cells) was subcutaneously administered to a CD8 deficient mouse (purchased from Jackson Laboratory) administered with CD1d expression enhanced B16 pulsed with α-GalCer, and the tumor resistance of these mice was compared.

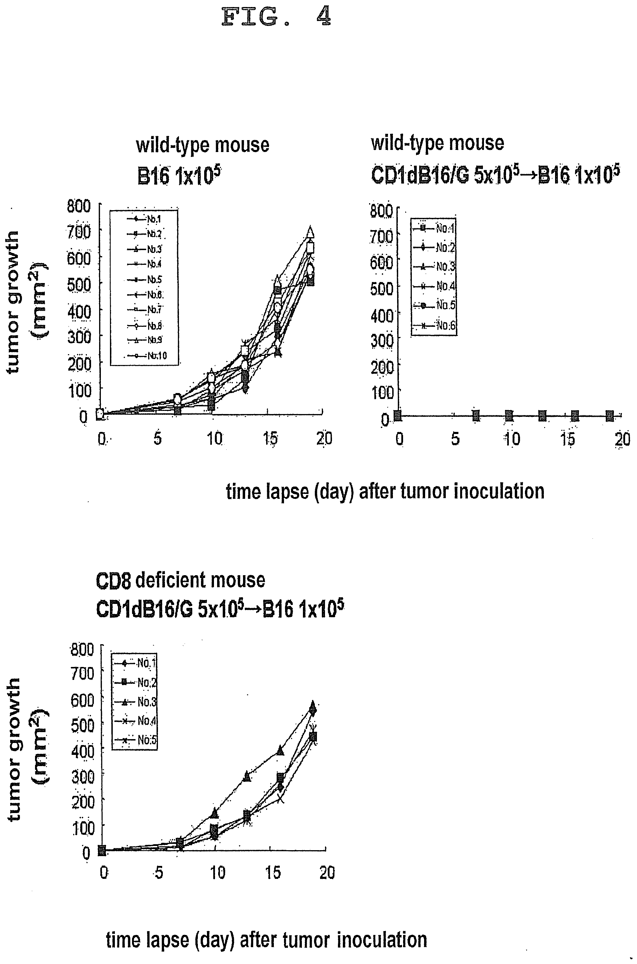

As a result, the wild-type mouse challenged with B16 showed resistance to the subcutaneously administered tumor. However, the tumor could not be eliminated in the CD8 deficient mouse (Fig. 4). This shows that administration of a tumor cell pulsed with a CD1d ligand such as α-GalCer leads to the function of CD8⁺ cytotoxic T lymphocytes (CTL) as an effecter cell, whereby an antitumor effect is provided.

From the foregoing, it has been clarified that the method developed by the present inventors can derive CTL to a tumor antigen expressed in the tumor.

### Example 5: Measurement of CD1d expression level in tumor cell line

To compare CD1d expression revels in various tumor cell lines, the CD1d expression levels in mouse-derived melanoma cell line B16, mouse-derived T lymphoma cell line EL4, mouse-derived plasma cell (B cell) line J558, mouse-derived monocytic leukemia cell line WEHI-3B, and these cell lines harboring a CD1d gene introduced by a retrovirus were measured by real time RT-PCR and flow cytometry.

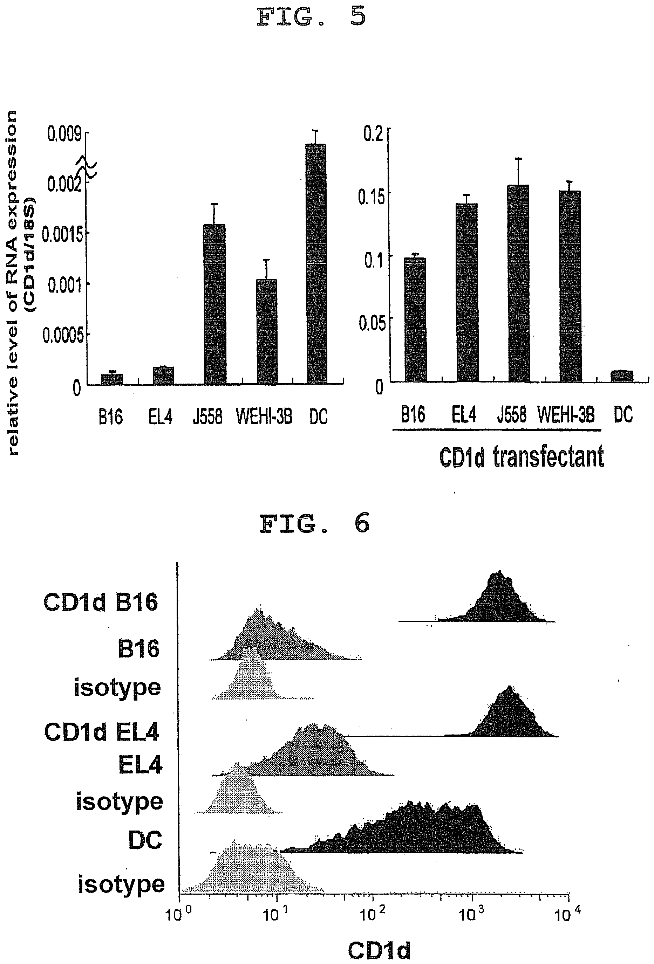

As a result, the expression of CD1d mRNA was confirmed in all tumor cell lines, and the expression of CD1d mRNA in tumor cell lines, into which CD1d gene had been introduced, showed a remarkable increase (Fig. 5). Furthermore, the expression of CD1d protein was examined in B16 and EL4 that showed relatively lower level of CD1d mRNA expression to find the expression of CD1d protein in these cell lines and remarkably enhanced expression levels due to the CD1d transgene (Fig. 6).

From the foregoing, it has been clarified that these tumor cells express CD1d protein and that the expression of CD1d protein is remarkably enhanced in a cell having an introduced CD1d gene.

### Example 6: CD1d-expressing tumor cell as target of activated NKT cells

Then, whether or not CD1d-expressing tumor cell can be a target of activated NKT cells was considered. To be specific, a tumor cell was pulsed with α-GalCer for 48 hr, and labeled with ⁵¹Cr. After washing, the obtained tumor cell was co-cultured with liver-derived mononuclear cells, and the amount of released ⁵¹Cr was measured, based on which the cytotoxic activity was measured.

As a result, CD1d forcibly expressing tumor cell pulsed with α-GalCer released a higher amount of ⁵¹Cr than a tumor cell not pulsed with α-GalCer. This shows that a tumor cell pulsed with a CD1d ligand activates NKT cells, and then is killed as a target of the NKT cells.

From the foregoing, it has been clarified that a cell pulsed with a CD1d ligand can be a target of NKT cells.

### Example 7: CD1d-expressing tumor cell as target of activated NKT cells

Then, whether or not CD1d-expressing tumor cell can be a target of activated NKT cells was considered. To be specific, CD1d forcibly expressing EL4 pulsed with α-GalCer was labeled with CFSE (carboxyfluorescein succinimidyl ester) and administered to a mouse. After 10 hr, splenocytes were collected from the mouse, and CFSE uptake by dendritic cell was measured using a flow cytometer.

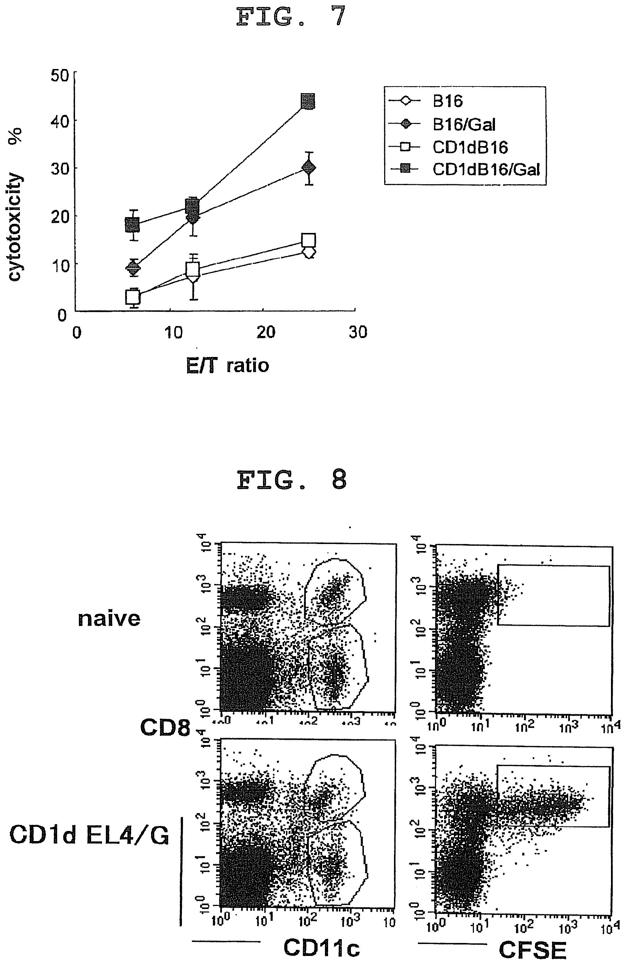

As a result, it has been confirmed that CD11c⁺ (particularly, CD11c⁺CD8a⁺) dendritic cell uptakes CFSE (Fig. 8) .

From the foregoing, it has been suggested that the administered tumor cell is killed by activation of NK/NKT cells, and the antigen is captured and presented by the adjacent dendritic cell, whereby the antitumor immunity can be achieved.

### (Conclusion)

NKT cells are a cell group connecting natural immunity and acquired immunity via maturation of dendritic cells. In this study, it has been demonstrated that when a CD1d ligand is presented by a CD1d-expressing tumor, strong in vivo activation of IFN-γ producing NKT cells and NK cells can be sustained, that the tumor cell is killed by the activation of NK/NKT cells, and the antigen is captured and presented by the adjacent dendritic cell, and that the acquired immunity to the antigen can be later induced. Once the acquired immunity is induced, tumor immunity is input in the memory. In this way, a CD1d-expressing tumor cell pulsed with a ligand can induce natural immunity in the short run and acquired immunity in the long run, which is considered a highly effective method for the tumor immunotherapy in the future.

Hence, the achievements of the studies by the present inventors and the items derived from the achievements are as follows.
1) The present inventors successfully activated NK/NKT cells in vivo by using a tumor cell pulsed with a CD1d ligand in vitro. Single administration of a CD1d ligand conventionally employed activates NK/NKT cells only temporarily. However, the present method activates these cells in a sustained manner, which in turn enables production of IFN-γ on a long-term basis.
2) The present method can induce cytotoxic T cells to the tumor antigen expressed in the tumor. Therefore, the method can establish acquired immunity and memory immunity to the tumor.
3) NK/NKT cells and T cells were successfully further activated by strong expression of a CD1d molecule in a tumor cell by transgene, and pulsing with a CD1d ligand.
4) The present method is highly convenient since it can be easily performed once a tumor cell is harvested.
5) According to the method developed by the present inventors, not only an immunotherapy using tumor cells of leukemia and the like, but also an immunotherapy using virus-infected cells that express specific antigen like tumor cells and the like become possible.

### Industrial Applicability

In the present invention, a target antigen and CD1d co-expressing cell acquires, after pulsing with a CD1d ligand, an immunity activating ability for a target antigen. The thus-obtained cell is useful for the prophylaxis or treatment or immunotherapy of neoplastic diseases (e.g., solid tumor containing epithelial tumor or nonepithelial tumor, leukemia, lymphoma, myeloma) or infections (e.g., virus infections). The production method of the present invention is useful for the preparation of such cells.

This application is based on a patent application No. 2006-045193 filed February 22, 2006 in Japan.

## Claims

1. A method of preparing an immunoinducer to a target antigen, comprising pulsing a target antigen and CD1d co-expressing cell with a CD1d ligand thereby presenting the CD1d ligand on the surface of the cell via CD1d, wherein the immunoinducer is for use in a method of prophylaxis or treatment of a neoplastic disease or infection and wherein the co-expressing cell is produced by:
(i) transforming a cell with a vector that expresses at least a target antigen or CD1d; or
(ii) introducing the target antigen and CD1d, such that the target antigen and CD1d will be co-expressed in the object cell, or expression of the target antigen and/or CD1d will be enhanced in the target antigen and CD1d co-expressing cell,
provided that the cell is not a dendritic cell.

2. An immunoinducer to a target antigen, comprising a target antigen and CD1d co-expressing cell, wherein the co-expressing cell has been pulsed with a CD1d ligand and the CD1d ligand is thereby presented on the surface of the cell via CD1d and wherein the co-expressing cell is produced by:
(i) transforming a cell with a vector that expresses at least a target antigen or CD1d; or
(ii) introducing the target antigen and CD1d, such that the target antigen and CD1d will be co-expressed in the object cell, or expression of the target antigen and/or CD1d will be enhanced in the target antigen and CD1d co-expressing cell,
wherein the immunoinducer is for use in a method of prophylaxis or treatment of a neoplastic disease or infection,
provided that the cell is not a dendritic cell.

3. A composition comprising the immunoinducer of claim 2, and an adjuvant.

4. Use of a target antigen and CD1d co-expressing cell capable of activating immunity to the target antigen, wherein the co-expressing cell has been pulsed with a CD1d ligand and the CD1d ligand is thereby presented on the surface of the cell via CD1d, in the manufacture of a medicament for use in a method of prophylaxis or treatment of a neoplastic disease or infection,
provided that the cell is not a dendritic cell,
and wherein the co-expressing cell is produced by:
(i) transforming a cell with a vector that expresses at least a target antigen or CD1d; or
(ii) introducing the target antigen and CD1d, such that the target antigen and CD1d will be co-expressed in the object cell, or expression of the target antigen and/or CD1d will be enhanced in the target antigen and CD1d co-expressing cell.

5. The use of claim 4, wherein the medicament comprises an adjuvant.

## Patentansprüche

1. Verfahren zum Herstellen eines Immuninduzierers für ein Zielantigen, umfassend ein Pulsieren einer Zielantigen und CD1d koexprimierenden Zelle mit einem CD1d-Liganden, wodurch der CD1d-Ligand auf der Oberfläche der Zelle über CD1d präsentiert wird, wobei der Immuninduzierer zur Verwendung in einem Verfahren zur Prophylaxe oder Behandlung einer neoplastischen Erkrankung oder Infektion dient, und wobei die koexprimierende Zelle hergestellt wird durch:
(i) Umwandeln einer Zelle mit einem Vektor, der mindestens ein Zielantigen oder CD1d exprimiert; oder
(ii) Einführen des Zielantigens und des CD1d, sodass das Zielantigen und das CD1d in der Objektzelle koexprimiert werden oder die Expression des Zielantigens und/oder des CD1d in der Zielantigen und CD1d koexprimierenden Zelle verbessert wird,
vorausgesetzt, die Zelle ist keine dendritische Zelle.

2. Immuninduzierer für ein Zielantigen, umfassend eine Zielantigen und CD1d koexprimierende Zelle, wobei die koexprimierende Zelle mit einem CD1d-Liganden gepulst wurde und der CD1d-Ligand dadurch auf der Oberfläche der Zelle über CD1d präsentiert wird, und wobei die koexprimierende Zelle hergestellt wird durch:
(i) Umwandeln einer Zelle mit einem Vektor, der mindestens ein Zielantigen oder CD1d exprimiert; oder
(ii) Einführen des Zielantigens und des CD1d, sodass das Zielantigen und das CD1d in der Objektzelle koexprimiert werden oder die Expression des Zielantigens und/oder des CD1d in der Zielantigen und CD1d koexprimierenden Zelle verbessert wird,
wobei der Immuninduzierer zur Verwendung in einem Verfahren zur Prophylaxe oder Behandlung einer neoplastischen Erkrankung oder Infektion dient,
vorausgesetzt, die Zelle ist keine dendritische Zelle.

3. Zusammensetzung, umfassend den Immuninduzierer nach Anspruch 2 und ein Adjuvans.

4. Verwendung einer Zielantigen und CD1d koexprimierenden Zelle, die fähig ist, Immunität gegen das Zielantigen zu aktivieren, wobei die koexprimierende Zelle mit einem CD1d-Liganden gepulst wurde und der CD1d-Ligand dadurch auf der Oberfläche der Zelle über CD1d präsentiert wird, bei der Herstellung eines Medikaments zur Verwendung in einem Verfahren zur Prophylaxe oder Behandlung einer neoplastischen Erkrankung oder Infektion,
vorausgesetzt, die Zelle ist keine dendritische Zelle,
und wobei die koexprimierende Zelle hergestellt wird durch:
(i) Umwandeln einer Zelle mit einem Vektor, der mindestens ein Zielantigen oder CD1d exprimiert; oder
(ii) Einführen des Zielantigens und des CD1d, sodass das Zielantigen und das CD1d in der Objektzelle koexprimiert werden oder die Expression des Zielantigens und/oder des CD1d in der Zielantigen und CD1d koexprimierenden Zelle verbessert wird.

5. Verwendung nach Anspruch 4, wobei das Medikament ein Adjuvans umfasst.

## Revendications

1. Procédé de préparation d'un immunoinducteur à un antigène cible, comprenant la pulsation d'un antigène cible et d'une cellule co-exprimant CD1d avec un ligand CD1d, présentant ainsi le ligand CD1d à la surface de la cellule via CD1d, dans lequel l'immunoinducteur étant destiné à être utilisé dans
un procédé de prophylaxie ou de traitement d'une maladie ou d'une infection néoplasique et dans lequel la cellule co-exprimant est produite par:
(i) la transformation d'une cellule avec un vecteur qui exprime au moins un antigène cible ou CD1d; ou
(ii) l'introduction de l'antigène cible et du CD1d, de telle sorte que l'antigène cible et le CD1d soient co-exprimés dans la cellule objet, ou que l'expression de l'antigène cible et / ou du CD1d soit renforcée dans l'antigène cible et la cellule co-exprimant CD1d,
à condition que la cellule ne soit pas une cellule dendritique.

2. Imunoinducteurà un antigène cible, comprenant un antigène cible et une cellule co-exprimant CD1d, dans lequel la cellule co-exprimant a été pulsée avec un ligand CD1d et le ligand CD1d est ainsi présenté à la surface de la cellule via le CD1d et dans lequel la cellule co-exprimant est produite par:
(i) la transformationd'une cellule avec un vecteur qui exprime au moins un antigène cible ou CD1d; ou
(ii) l'introduction de l'antigène cible et du CD1d, de telle sorte que l'antigène cible et le CD1d soient co-exprimés dans la cellule objet, ou que l'expression de l'antigène cible et / ou du CD1d soit renforcée dans l'antigène cible et la cellule co-exprimant CD1d,
dans lequel l'immunoinducteur est destiné à être utilisé dans un procédé de prophylaxie ou de traitement d'une maladie ou d'une infection néoplasique,
à condition que la cellule ne soit pas une cellule dendritique.

3. Composition comprenant l'immunoinducteur de la revendication 2 et un adjuvant.

4. Utilisation d'un antigène cible et d'une cellule co-exprimant CD1d capable d'activer l'immunité vis-à-vis de l'antigène cible, la cellule co-exprimant ayant été pulsée avec un ligand CD1d et le ligand CD1d étant ainsi présenté à la surface de la cellule via CD1d, dans la fabrication d'un médicament destiné à être utilisé dans une méthode de prophylaxie ou de traitement d'une maladie ou d'une infection néoplasique,
à condition que la cellule ne soit pas une cellule dendritique,
et dans lequel la cellule de co-expression est produite par:
(i) la transformation d'une cellule avec un vecteur qui exprime au moins un antigène cible ou CD1d; ou
(ii) l'introduction de l'antigène cible et du CD1d, de telle sorte que l'antigène cible et le CD1d soient co-exprimés dans la cellule objet, ou que l'expression de l'antigène cible et / ou du CD1d soit renforcée dans l'antigène cible et la cellule co-exprimant CD1d.

5. Utilisation selon la revendication 4, dans laquelle le médicament comprend un adjuvant.
